# EUROPÄISCHE PATENTSCHRIFT

(11) **EP 2 318 079 B1**
(45) Veröffentlichungstag und Bekanntmachung des Hinweises auf die Patenterteilung: **29.05.2013**
(21) Anmeldenummer: 09782237.3
(22) Anmeldetag: 26.08.2009
(51) Int. Cl.: A61M 15/00, G06M 1/04

(54) **ANTRIEBSEINHEIT FÜR DOSISZÄHLER**
DRIVE UNIT FOR DOSAGE COUNTER
UNITÉ D'ENTRAÎNEMENT POUR UN COMPTEUR DE DOSE

(30) Priorität: 28.08.2008 DE 102008044770
(43) Veröffentlichungstag der Anmeldung: 11.05.2011
(73) Patentinhaber: Boehringer Ingelheim International GmbH, 55216 Ingelheim am Rhein (DE)
(72) Erfinder: HOCHRAINER, Dieter, 55216 Ingelheim am Rhein (DE)
(74) Vertreter: Simon, Elke Anna Maria
(86) Internationale Anmeldenummer: PCT/EP2009/061023
(87) Internationale Veröffentlichungsnummer: WO 2010/023233

(56) Entgegenhaltungen:
- WO-A-03/107269
- WO-A-2006/051073
- DE-A1-102006 049 614
- US-A1- 2004 144 798

## Beschreibung

Die vorliegende Erfindung betrifft einen Zerstäuber gemäß dem Oberbegriff des Anspruchs 1.

Die vorliegende Erfindung betrifft vorzugsweise Zerstäuber in Form von Inhalatoren. Insbesondere bezieht sich die Erfindung hierbei auf sogenannte Metered Dose Inhalers (MDIs), d. h. auf Inhalatoren, bei denen eine flüssige Arzneimittelformulierung aus einem unter Druck stehenden Behälter (Aerosol-Behälter) insbesondere mittels eines Dosierventils dosiert als Aerosol ausgegeben bzw. vernebelt wird. Jedoch kann die vorliegende Erfindung auch bei sonstigen Inhalatoren oder Zerstäubern eingesetzt werden, bei denen es auf eine Zählung der ausgegebenen Dosen ankommt.

Die WO 00/09187 A1, die den Ausgangspunkt bildet, offenbart einen MDI mit einer Zähleinrichtung zur Zählung von Betätigungen und/oder von abgegebenen Aerosol-Dosen. Bei Betätigung wird ein die auszugebende Arzneimittelformulierung enthaltender Behälter in ein Gehäuse des MDIs gedrückt. Diese Hubbewegung wird von der Zähleinrichtung erfaßt und treibt einen Zählring an. Die Zähleinrichtung weist hierzu ein kniehebelartiges, elastisch verformbares Antriebselement auf. Das Antriebselement wird bei Betätigung des MDIs gleichzeitig sowohl axial als auch in Drehrichtung (Umfangsrichtung) des Zählrings verlagert, um den Zählring weiterzudrehen.

Die elastische Verformung des Antriebselements bei dem bekannten MDI kann problematisch sein. Insbesondere ist eine einwandfreie Rückstellung des Antriebselements unbedingt erforderlich, wenn eine einwandfreie Funktion der Zähleinrichtung gewährleistet sein soll. Dies erfordert sehr geringe Herstellungstoleranzen.

Bei dem bekannten MDI ist des weiteren problematisch bzw. nachteilig, daß ein unvollständiges Betätigen - also bei nur teilweisem Hub des Behälters - kein sicheres Weiterzählen der Zähleinrichtung bzw. des Zählrings gewährleistet ist, auch wenn trotz teilweiser Betätigung eine Dosis ausgegeben wird.

Weitere bekannte Inhalatoren bzw. Schrittschaltwerke werden in folgenden Dokumenten beschrieben:

Die WO 03/107269 A1 betrifft ein Zählwerk zum Zählen dosierter Abgaben flüssiger Produkte und umfasst im Wesentlichen einen Zählring, eine Schaltvorrichtung und ein in Richtung der Längsachse bewegbares Betätigungsmittel. Die Schaltvorrichtung wandelt die Linearbewegung des Betätigungsmittels in eine Drehung des Zählrings um und umfasst ein Führungselement und ein Schaltelement. Das Führungselement enthält eine Kurvenfläche, durch die ein Vorsprung am Schaltelement geführt wird.

Die WO 2006/051073 A1 und die DE 10 2006 049614 A1 zeigen Schrittschaltwerke für Handgeräte zur portionierten Ausgabe von Inhaliermedikamenten. Die Schrittschaltwerke enthalten eine Narbe mit dünnen federnden Schaltfingern, gegen die linear mitgeschleppt von der Betätigungsbewegung am Handgerät ein Planetenradgetriebe mit Skalenring vertikal verlagert wird. Bei dieser Verlagerung vollzieht die Narbe mit Hilfe von mantelseitigen schrägen Sehlitzen ein Drehaufgleiten relativ zum Planetenradgetriebe. Dadurch treiben die Schaltfinger durch Eingriff von unten in eine gezahnte Scheibe das Sonnenrad des Planetengetriebes schrittweise an.

Die US 2004/144798 A1 betrifft einen Dosiszähler, der unterhalb eines zur Dosis-Abgabe bewegten Behälters angeordnet ist. Der Dosiszähler umfasst einen Zählring und ein Antriebselement, Das Antriebselement besteht aus einem flachen Ring mit nach oben und unten gerichteten flexiblen Zähnen. Durch Bewegung des Behälters nach unten, werden sowohl die nach oben als auch die nach unten gerichteten Zähne zusammengedrückt, wobei die unteren Zähne den Zählring durch Eingriff in eine Zahnung an seiner Oberseite drehen.

Der vorliegenden Erfindung liegt die Aufgabe zugrunde, einen Zerstäuber mit einer verbesserten Zähleinrichtung anzugeben.

Die obige Aufgabe wird durch einen Zerstäuber gemäß Anspruch 1 gelöst. Vorteilhafte Weiterbildungen sind Gegenstand der Unteransprüche.

Die vorliegende Erfindung liegt darin, dass die Zähleinrichtung einen Zählring und eine Antriebseinrichtung mit einem dem Zählring zugeordneten ringförmigen und starren Antriebselement zum Antreiben des Zählrings oder eines dem Zählring zugeordneten Zahnrads aufweist, wobei das Antriebselement mit einer axialen Zahnung ausgebildet ist und eine erste Führungsbahn die eine schiefe Ebene bildet oder enthält oder von einem Schraubenbahnabschnitt gebildet wird und dass die Antriebseinrichtung ein der Führungsbahn zugeordnetes Führungselement zur Umwandlung einer Axialbewegung in eine Drehbewegung zum Antrieb des Zählrings oder Zahnrads aufweist.

Gemäß einer nicht beanspruchten Ausführungsform

ist der Zerstäuber oder die Zähleinrichtung derart ausgebildet, dass bereits eine nur teilweise Betätigung des Zerstäubers zum Weiterzählen des Zählwerks genügt. Dies führt zu einer besonders funktionssicheren Zählung und damit sehr funktionssicheren Benutzung des Zerstäubers.

Weitere Vorteile, Merkmale, Eigenschaften und Aspekte der vorliegenden Erfindung ergeben sich aus der folgenden Beschreibung einer bevorzugten Ausführungsform anhand der Zeichnung. Es zeigt:
- Fig. 1: einen schematischen Schnitt eines vorschlagsgemäßen Zerstäubers mit einer Zähleinrichtung;
- Fig. 2: eine explosionsartige Darstellung von Teilen der Zähleinrichtung;
- Fig. 3: einen schematischen Teilschnitt der Zähleinrichtung ohne Gehäuse bei betätigtem Zerstäuber; und
- Fig. 4: eine schematische Darstellung in der Art einer Abwicklung einer Antriebseinrichtung der Zähleinrichtung bei anfänglicher Betätigung des Zerstäubers.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

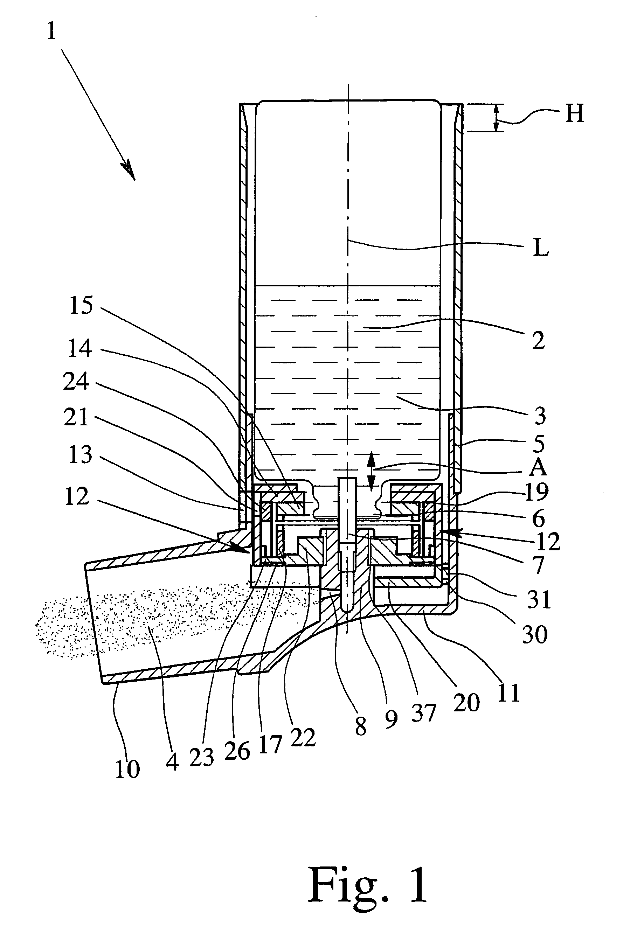

Fig. 1 zeigt in einem nur schematischen Schnitt eine bevorzugte Ausführungsform eines Zerstäubers 1 gemäß der vorliegenden Erfindung. Bei dem Zerstäuber 1 handelt es sich insbesondere um einen Inhalator, vorzugsweise einen MDI.

Insbesondere ist der Zerstäuber 1 zur Ausgabe einer Arzneimittelformulierung bzw. eines Fluids 2 aus einem Behälter 3 als Sprühnebel bzw. Aerosol 4 ausgebildet, wie schematisch in Fig. 1 angedeutet. Hierbei ist anzumerken, daß Fig. 1 den Zerstäuber 1 im nicht betätigten Zustand zeigt, die Sprühwolke bzw. das Aerosol 4 jedoch aus Veranschaulichungsgründen angedeutet ist.

Der Zerstäuber 1 weist beim Darstellungsbeispiel ein Gehäuse 5 auf, in das der Behälter 3 vorzugsweise eingesetzt bzw. einsetzbar ist. Jedoch sind hier auch andere konstruktive Lösungen möglich.

Der Zerstäuber 1 oder Behälter 3 weist vorzugsweise ein Ventil 6, insbesondere ein Dosierventil, auf. Besonders bevorzugt weist das Ventil 6 ein insbesondere zum Öffnen des Ventils 6 bewegbares Ventilelement, insbesondere einen axial eindrückbaren Ventilstamm 7, auf. Jedoch sind hier auch andere konstruktive Lösungen möglich.

Der Zerstäuber 1 weist vorzugsweise eine Sprüheinrichtung oder Düse 8 auf, die an den Behälter 3 bzw. das Ventil 6 oder dessen Ventilelement bzw. Ventilstamm 7 - beim Darstellungsbeispiel über ein Anschlußstück 9 - zur sprühenden Ausgabe des Fluids 2 bzw. Bildung des Aerosols 4 bei Betätigung des Zerstäubers 1 angeschlossen ist. Vorzugsweise ist die Düse 8 vom Anschlußstück 9 gebildet, insbesondere in einer Seitenfläche des vorzugsweise im wesentlichen hohlzylindrischen Anschlußstücks 9. Jedoch sind hier auch andere konstruktive Lösungen möglich.

Der Zerstäuber 1 weist vorzugsweise ein Mundstück 10 oder sonstiges Endstück zur Ausgabe des Aerosols 4 auf, das besonders bevorzugt durch ein als Winkelstück ausgebildetes Gehäuseteil 11 gebildet oder gehalten ist. Jedoch sind hier auch andere konstruktive Lösungen möglich.

Der Zerstäuber 1 bzw. dessen Gehäuse 5 und/oder der Behälter 3 ist bzw. sind vorzugsweise länglich ausgebildet. Die Ausgabe des Aerosols 4 erfolgt vorzugsweise geneigt oder quer zu dieser Längsrichtung L. Das Mundstück 10 bzw. sonstige Endstück ist vorzugsweise dementsprechend abgewinkelt oder abklappbar, gegebenenfalls auch verstellbar, insbesondere verschwenkbar.

Bei Betätigung des Zerstäubers 1 wird das Ventil 6 geöffnet und insbesondere nur eine Dosis des Fluids 2 über den Ventilstamm 7, das Anschlußstück 9 und die Düse 8 ausgegeben. In der Düse 8 folgt eine Zerstäubung bzw. Vernebelung als Aerosol 4, das über das Mundstück 10 oder sonstige Endstück ausgegeben wird. Das Aerosol 4 kann dann inhaliert werden.

Die Betätigung des Zerstäubers 1 bzw. das Öffnen des Ventils 6 erfolgt vorzugsweise durch axiales Eindrücken des Ventilstamms 7 oder durch sonstige Betätigung des Ventils 6.

Beim Darstellungsbeispiel wird bei der bzw. zur Betätigung des Zerstäubers 1 der Behälter 3 bzw. das Ventil 6 einerseits relativ zum Anschlußstück 9 bzw. Gehäuseteil 11 andererseits zueinander bewegt bzw. zusammengedrückt, insbesondere in Richtung der Längsachse L in einer Axialbewegung, wie durch Pfeil A angedeutet. Beim Darstellungsbeispiel erfolgt dies dadurch, daß der Behälter 3 bodenseitig (bei Fig. 1 oben) weiter in das offene Gehäuse 5 manuell - insbesondere gegen die Federkraft des Ventils 6 und/oder eines sonstigen Federelements - eingedrückt wird. Jedoch ist die Betätigung beispielsweise auch umgekehrt durch Drücken des auf das Gehäuseteil 11 (Winkelstück) oder ein sonstiges Betätigungsteil möglich, wobei dann das Gehäuse 5 beispielsweise oben geschlossen und das Gehäuseteil 11 oder sonstige Teil relativ zum Gehäuse 5 verschiebbar, betätigbar oder eindrückbar ist.

Zur Betätigung des Zerstäubers 1 sind jedoch auch andere konstruktive Lösungen möglich.

Durch die Axialbewegung A öffnet das Ventil 6, und eine Dosis des Fluids 2 wird ausgegeben. Bei vollständiger Betätigung des Zerstäubers 1, also vollständiger Axialbewegung A, erfolgt ein Hub H des Behälters 3 oder eines sonstigen Betätigungsteils des Zerstäubers - wie des Gehäuseteils 11, des Anschlußstücks 9, des Ventilelements, wie des Ventilstamms 7, oder dergleichen- insbesondere relativ zum Ventil 6 oder Gehäuse 5.

Je nach Konstruktion des Zerstäubers 1 bzw. Ventils 6 erfolgt auch bei nicht vollständigem Hub H - also bei unvollständiger Betätigung - oftmals die Ausgabe des Aerosols 4, also die Ausgabe einer Dosis des Fluids 2.

Der Zerstäuber 1 weist eine Zähleinrichtung 12 zum Zählen von Betätigungen des Zerstäubers 1 bzw. von abgegebenen Aerosol-Dosen auf. Die Zähleinrichtung 12 ist in Fig. 1 schematisch im Schnitt dargestellt. Sie ist vorzugsweise im oder am Gehäuse 5, insbesondere Kopfstück, Winkelteil oder Gehäuseteils 11, und/oder am Kopf des Behälters 3 angeordnet. Jedoch sind hier auch andere konstruktive Lösungen möglich.

Zählwerte der Zähleinrichtung 12 sind über ein Sichtfenster 13, das hier im Gehäuse 5 und/oder im Gehäuseteil 11 gebildet ist, anzeigbar bzw. ablesbar.

Die Zähleinrichtung 12 kann beispielsweise die noch vorhandenen Aerosol-Dosen oder die bereits erfolgten Betätigungen bzw. ausgegebenen Aerosol-Dosen als Zählwert anzeigen.

Die Zähleinrichtung 12 weist vorzugsweise mindestens einen Zählring 14 und/oder ein zugeordnetes Zahnrad 15 auf.

Gemäß einer Ausführungsvariante kann das Zahnrad 15 auch einen ersten Zählring und der Zählring 14 einen zweiten Zählring bilden. Insbesondere dann ist das Zahnrad 15 vorzugsweise über eine nicht dargestellte Untersetzung mit dem Zählring 14 gekoppelt, insbesondere so, daß nach einer bestimmten Anzahl von Zählschritten des Zahnrads 15 dann der Zählring 14 um einen Zählschritt weitergedreht bzw. -bewegt wird.

Wenn nur ein Zählring 14 oder darüber hinaus noch ein zusätzlicher nicht dargestellter Zählring vorgesehen ist, kann das Zahnrad 15 wahlweise mit dem Zählring 14 unmittelbar oder über eine Untersetzung gekoppelt sein oder bedarfsweise sogar direkt vom Zählring 14 gebildet oder einstückig mit diesem ausgebildet sein.

Die vorgenannten Varianten sollen besonders bevorzugt durch die Beschreibung bzw. Bezeichnung "das Zahnrad 15 ist dem Zählring 14 zugeordnet" mit abgedeckt sein.

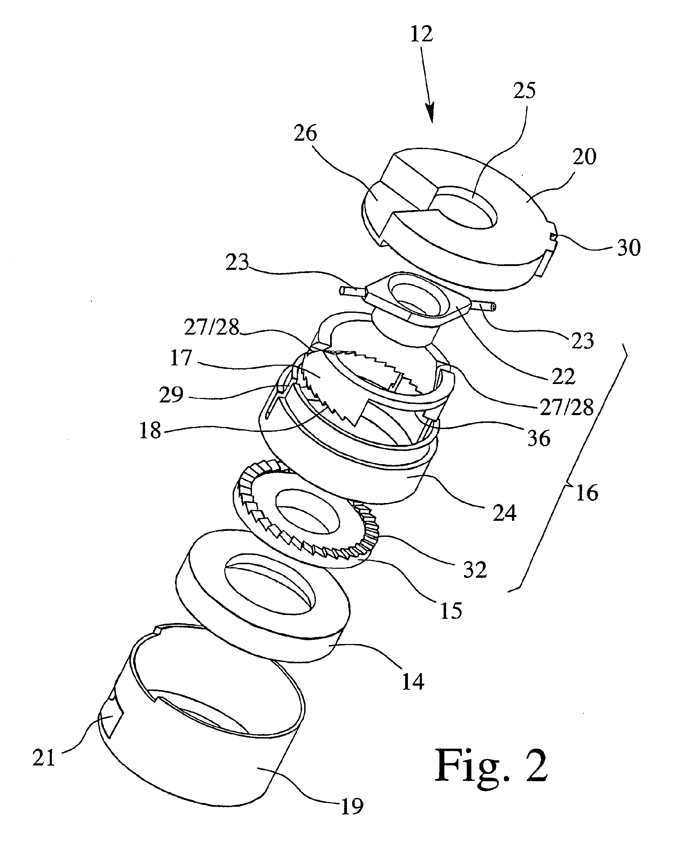

Die Zähleinrichtung 12 weist weiter eine Antriebseinrichtung 16 zum Antreiben, insbesondere schrittweisen Weiterdrehen, des Zählrings 14 oder Zahnrads 15 auf, die in Fig. 2 explosionsartig dargestellt ist.

Die Antriebseinrichtung 16 weist ein Antriebselement 17 auf, das dem Zählring 14 bzw. Zahnrad 15 zum schrittweisen Weiterdrehen zugeordnet ist.

Vorzugsweise ist das Antriebselement 17 zumindest im wesentlichen ringförmig, mit einer axialen Zahnung 18 und/oder starr ausgebildet. Die Zahnung 18 korrespondiert mit der Zahnung 32 des Zahnrads 15, wobei nicht auf der gesamten Unterseite des Antriebselements 17 und/oder nicht auf der gesamten Oberfläche des Zahnrads 15 jeweils Zähne vorhanden sein müssen. Ggf. können auch nur einzelne Zähne vorhanden sein.

Insbesondere ist das Antriebselement 17 zunächst zum Zählring 14 bzw. Zahnrad 15 hin verlagerbar und besonders bevorzugt erst anschließend gemeinsam mit diesem drehbar.

Vorzugsweise ist der Zerstäuber 1 bzw. die Zähleinrichtung 12 bzw. deren Antriebseinrichtung 16 derart ausgebildet, daß bereits eine nur teilweise Betätigung des Zerstäubers 1 bzw. nur ein teilweiser Hub H zum Weiterzählen der Zähleinrichtung 12 bzw. Weiterdrehen des Zahnrads 15 oder Zählrings 14 um einen Zählschritt genügt. Eine mögliche konstruktive Lösung ergibt sich aus der noch folgenden näheren Beschreibung eines bevorzugten Aufbaus der Zähleinrichtung 12.

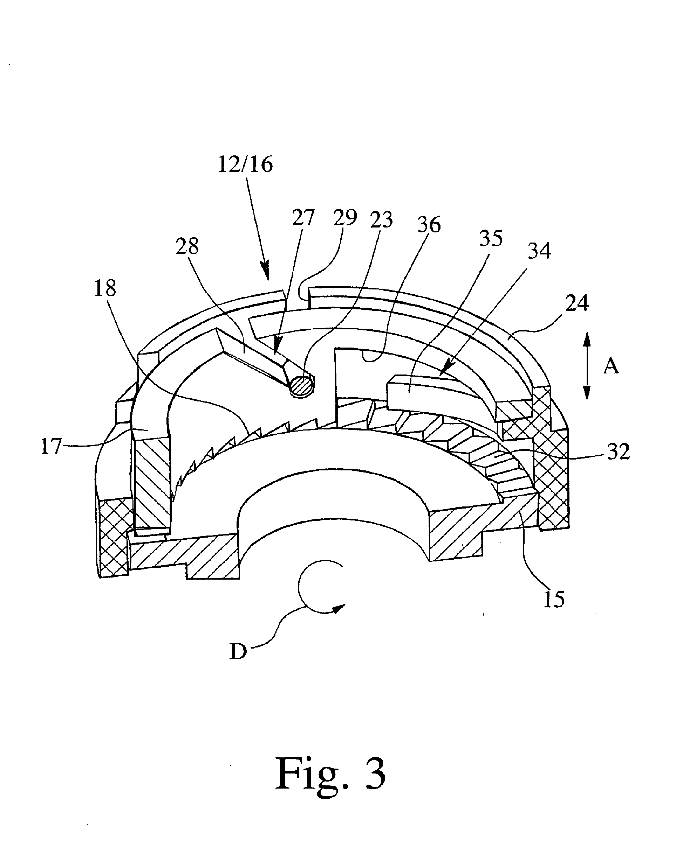

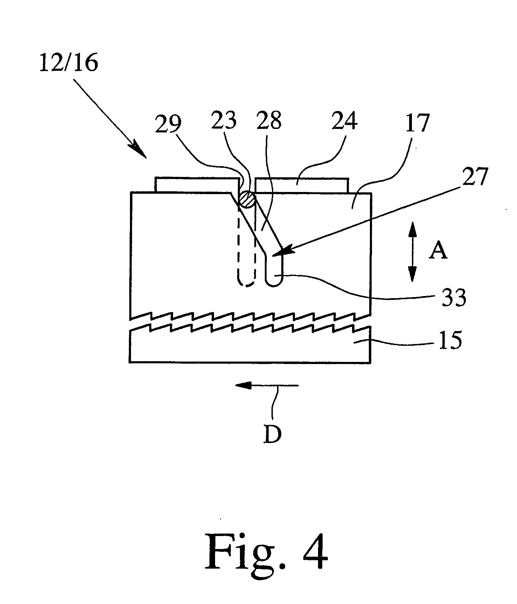

Zur Erläuterung eines bevorzugten Aufbaus der Zähleinrichtung 12 und/oder der bevorzugten Funktion der Zähleinrichtung 12 wird nachfolgend ergänzend zu Fig. 1 auch auf die Fig. 2 bis 4 Bezug genommen. Fig. 2 zeigt Komponenten bzw. Teile der Zähleinrichtung 12 in einer explosionsartigen Darstellung. Fig. 3 zeigt in einem schematischen Teilschnitt ausschnittsweise die Zähleinrichtung 12 bei betätigtem Zerstäuber 1. Fig. 4 zeigt in einer schematischen Abwicklung Teile der Zähleinrichtung 12 bzw. Antriebseinrichtung 16 in einem anfänglichen Stadium der Betätigung des Zerstäubers 1.

Die Zähleinrichtung 12 weist vorzugsweise ein Zählwerksgehäuse auf, das hier aus einem Unterteil 19 und einem Oberteil 20 gebildet ist. Die Begriffe "Unterteil" und "Oberteil" werden nur zur leichteren Beschreibung gewählt, sagen jedoch nichts über die Orientierung bei Benutzung des Zerstäubers 1 bzw. der Zähleinrichtung 12 aus. Weiter sind auch andere konstruktive Lösungen möglich.

Im Zählwerksgehäuse - beim Darstellungsbeispiel im Unterteil 19 - ist vorzugsweise ein Sichtfenster 21 zur Anzeige bzw. Ablesbarkeit von Zählwerten der Zähleinrichtung 12 vorgesehen. Das Sichtfenster 21 ist insbesondere dem Fenster 13 zugeordnet. Beispielsweise kann es sich bei dem Sichtfenster 21 um eine Durchbrechung oder Öffnung handeln, insbesondere wenn das Fenster 13 zwar durchsichtig, aber durch eine Wandung geschlossen ist. Es ist auch umgekehrt möglich, daß das Sichtfenster 21 durch eine Wandung, Abdeckung oder dergleichen verschlossen, jedoch durchsichtig ist.

Die Zähleinrichtung 12 weist weiter vorzugsweise ein Führungsteil 22 mit mindestens einem Führungselement 23, insbesondere zwei auf gegenüberliegenden Seiten angeordneten Führungselementen 23, sowie vorzugsweise eine Führungshülse 24 auf.

Die Zählwerkseinrichtung 12 wird vorzugsweise derart in den Zerstäuber 1 bzw. das Gehäuse 5 oder Gehäuseteil 11 eingebaut, daß es am Behälter 3 bzw. an einem sogenannten Ventilteller des Behälters 3/Ventils 6 - beim Darstellungsbeispiel mit dem Unterteil 19 - anliegt und/oder das Ventilelement, wie den Ventilstamm 7, aufnimmt oder umgibt. Das Anschlußstück 9 erstreckt sich vorzugsweise durch eine zentrale Öffnung 25 im Oberteil 20 in das Zählwerksgehäuse bis zum Ventil 6 bzw. Ventilstamm 7. Insbesondere bildet das Anschlußstück 9 eine Ventilstammaufnahme und weist hierzu eine zentrale Bohrung, Ausnehmung oder dergleichen mit vorzugsweise daran angeschlossener Düse 8 auf.

Bei Betätigung des Zerstäubers 1 führt die Axialbewegung A dazu, daß das Anschlußstück 9 tiefer in das Zählwerksgehäuse eintaucht, insbesondere daß das Unterteil 19 und Oberteil 20 relativ zum Anschlußstück 9 entlang der Längsachse L - bei der Darstellung gemäß Fig. 1 nach oben - verschoben werden. Das Zählwerksgehäuse bzw. Oberteil 20 weist beim Darstellungsbeispiel vorzugsweise eine sektorartige, periphere Vertiefung 26 auf, die sich in Umfangsrichtung radial nach außen hin erweitert, um sicherzustellen, daß die Düse 8 auch bei betätigtem Zerstäuber 1, wenn das Anschlußstück 9 tiefer in dem Oberteil 20 sitzt, das Aerosol 4 in gewünschter Weise seitlich in Richtung des Mundstücks 10 oder sonstigen Endstücks ausgeben kann. Jedoch sind hier auch andere konstruktive Lösungen möglich.

Das Führungsteil 22 paßt vorzugsweise auf das Anschlußstück 9. Vorzugsweise durchgreift das Anschlußstück 9 das Führungsteil 22. Besonders bevorzugt ist das Führungsteil 22 am Anschlußstück 9 axial widergelagert, so daß bei der Axialbewegung A bzw. beim Betätigen des Zerstäubers 1 das Führungsteil 22 nicht axial weiter auf das Anschlußstück 9 aufschiebbar ist. Dies kann beispielsweise durch einen entsprechenden axialen Anschlag, wie eine in Fig. 1 nur schematisch angedeutete Schulter 37, realisiert werden.

Statt am Anschlußstück 9 kann das Führungsteil 22 auch an einem sonstigen Teil des Zerstäubers 1, insbesondere dem Gehäuseteil 11, widergelagert sein.

Die Führungselemente 23 erstrecken sich vorzugsweise radial vom Führungsteil 22 nach außen, insbesondere auf entgegengesetzten Seiten.

Die Führungselemente 23 sind vorzugsweise zapfenartig ausgebildet.

Wenn nachfolgend immer nur von einem Führungselement 23 gesprochen wird, ist dies dadurch bedingt, daß für die Funktion grundsätzlich auch ein einziges Führungselement 23 genügt, auch wenn vorzugsweise zwei Führungselemente 23 auf entgegengesetzten Seiten aus konstruktiven Gründen, Stabilitätsgründen und/oder zur Sicherheit vorgesehen sind.

Die Zähleinrichtung 12 bzw. Antriebseinrichtung 16 weist vorzugsweise eine erste Führungsbahn 27 und/oder eine schiefe Ebene 28 für das Führungselement 23, beim Darstellungsbeispiel sogar zwei erste Führungsbahnen 27 bzw. schiefe Ebenen 28 für die beiden Führungselemente 23, auf.

Die erste Führungsbahn 27 bzw. schiefe Ebene 28 ist jeweils vorzugsweise im oder vom Antriebselement 17 gebildet. Jedoch sind auch andere konstruktive Lösungen möglich.

Die Führungselemente 23 durchgreifen ihre zugeordnete erste Führungsbahn 27 jeweils vorzugsweise radial und sind mit ihren freien Enden jeweils vorzugsweise in zweiten Führungsbahnen 29 gerührt, die vorzugsweise in bzw. von der Führungshülse 24 gebildet sind.

Vorzugsweise verlaufen die zweiten Führungsbahnen 29 nur in axialer Richtung. Dementsprechend sind die Führungselemente 23 jeweils nur in axialer Richtung bezüglich der Drehachse des Zählrings 14 bzw. Zahnrads 15 verschieblich geführt. Jedoch sind hier auch andere konstruktive Lösungen möglich.

Je nach Konstruktion sind die Führungsbahnen 27 und/oder 29 wahlweise als Schlitz, Nut oder dergleichen ausgebildet.

Die Führungshülse 24 ist vorzugsweise drehfest in das Zählwerksgehäuse eingebaut. Das Zählwerksgehäuse ist seinerseits vorzugsweise drehfest in den Zerstäuber 1 eingebaut. Hierzu weist das Oberteil 20 beispielsweise eine außenseitige Axialnut 30 auf, in die ein radialer und/oder stegartiger Vorsprung 31 bzw. eine Feder des Zerstäubers 1 bzw. Gehäuses 5 bzw. Gehäuseteils 11 eingreift, wie in Fig. 1 angedeutet, um eine drehfeste, aber verschiebbare Verbindung zum Zählwerksgehäuse herzustellen. Jedoch sind hier auch andere konstruktive Lösungen möglich.

Das Zahnrad 15 ist mit einer vorzugsweise axialen Zahnung 32 versehen, die insbesondere als Sägezahnung ausgeführt ist und/oder zu der Zahnung 18 des Antriebselements 17 derart korrespondiert, daß im axial zusammengerückten Zustand das Antriebselement 17 das Zahnrad 15 zumindest oder nur in einer Drehrichtung D (der Zählrichtung), wie in Fig. 3 angedeutet, antreiben bzw. weiter- oder mitdrehen kann.

In den Figuren nicht dargestellte Nummern bzw. Zählwerte sind vorzugsweise auf der peripheren Umfangsfläche oder einem sonstigen geeigneten Bereich des Zählrings 14 und/oder Zahnrads 15 angeordnet und durch die Fenster 13, 21 von außen sichtbar, um den jeweiligen Zählerstand der Zähleinrichtung 12 anzuzeigen.

Die Führungsbahn 27 oder 29 oder die schiefe Ebene 28 bildet vorzugsweise einen Schlitz oder eine Nut, in den bzw. die das zugeordnete Führungselement 23 eingreift. Jedoch sind auch andere konstruktive Lösungen möglich.

Fig. 4 veranschaulicht das Funktionsprinzip der vorschlagsgemäßen Antriebseinrichtung 16 der Zähleinrichtung 12.

Die Führungselemente 23 sind jeweils in der zweiten Führungsbahn 29 bezüglich der Drehachse des Zählrings 14 / Zahnrads 15 nur axial verschiebbar geführt, das Führungsteil 22 ist also gegen Verdrehen gesichert. Alternativ oder zusätzlich kann das Führungsteil 22 beispielsweise auch direkt am Gehäuseteil 11 bzw. Anschlußstück 9 und/oder auf sonstige Weise verdrehsicher gehalten sein.

Bei nicht betätigtem Zerstäuber 1 ist das Antriebselement 17 vorzugsweise von dem zugeordneten Zählrad 14 bzw. Zahnrad 15 axial abgerückt. Bei Betätigung des Zerstäubers 1 erfolgt die Axialbewegung A der Führungselemente 23 zum Zählring 14 bzw. Zahnrad 15 hin. Zu Beginn dieser Axialbewegung, die in Fig. 4 angedeutet ist, wird das Antriebselement 17 zunächst zum Zählring 14 bzw. Zahnrad 15 hin axial verschoben, so daß die Zahnungen 18 und 32 miteinander in Eingriff gebracht werden. Dies erfolgt dadurch, daß die Führungselemente über die ersten Führungsbahnen 27 auf das Antriebselement 17 einwirken, insbesondere eine kulissenartige Führung bzw. Zwangsführung bilden. Vorzugsweise wirken die Führungselemente 23 mit den schiefen Ebenen 28 derart zusammen, daß bei der anfänglichen Axialbewegung A zunächst das Antriebselement 17 nur oder im wesentlichen zum Zahnrad 15 hin axial verschoben wird, bis der Zahneingriff erfolgt.

Im Verlauf der weiteren Axialbewegung A der Führungselemente 23 wird dann das Antriebselement A zusammen mit dem durch das Kämmen der Zahnungen 18 und 32 mitdrehenden Zahnrad 15 in die Drehrichtung D gedreht.

Allgemeiner ausgedrückt erfolgt also eine Umwandlung der Axialbewegung A in die Drehbewegung D zum Antrieb des Zählrings 14 bzw. Zahnrads 15 mittels der ersten Führungsbahn 27 und/oder der schiefen Ebene 28 und einem zugeordneten Führungselement 23 und/oder durch eine kulissenartige Führung.

Vorzugsweise bildet die schiefe Ebene 28 einen Teil der ersten Führungsbahn 27. Besonders bevorzugt ist die schiefe Ebene 28 hier durch einen Schraubenbahnabschnitt gebildet. Der Schraubenbahnabschnitt bildet vorzugsweise wiederum einen Teil der ersten Führungsbahn 27.

Besonders bevorzugt ist der Zerstäuber 1 oder die Zähleinrichtung 12 bzw. deren Antriebseinrichtung 16 derart ausgebildet, daß bereits eine nur teilweise Betätigung des Zerstäubers 1, also eine nur teilweise Axialbewegung A, und insbesondere nur die anfängliche Axialbewegung A, zum Weiterzählen der Zählwerkeinrichtung 12, also Weiterdrehen des Zählrings 14 bzw. Zahnrads 15 um einen Zählwert genügt. Beim Darstellungsbeispiel wird dies dadurch erreicht, daß die erste Führungsbahn 27 bzw. die schiefe Ebene 28 derart ausgelegt ist, daß sich an den geneigten Abschnitt bzw. die schiefe Ebene 28 optional ein axial verlaufender Abschnitt 33 der Führungsbahn 27 anschließt. So kann erreicht werden, daß nach der anfänglichen Axialbewegung A im Verlauf der weiteren Axialbewegung A das Antriebselement 17 und damit auch das Zählrad 14 bzw. Zahnrad 15 nicht mehr weitergedreht werden. So wird erreicht, daß nur die anfängliche Axialbewegung A das Weiterdrehen und damit Weiterzählen bewirkt. Dementsprechend kann auch bei einer unvollständigen Betätigung des Zerstäubers 1, insbesondere bei Nichterreichen des maximalen Hubs H, korrekt weitergezählt werden, da auch in diesem Fall üblicherweise eine Aerosol-Dosis vom Zerstäuber 1 abgegeben wird.

Beim Darstellungsbeispiel verlaufen der Schlitz bzw. die Nut der ersten Führungsbahn 27 ausgehend von einer in Fig. 4 dargestellten Eingriffsposition des Führungselements 23 bei nicht betätigtem Zerstäuber 1 aus zunächst geneigt und dann axial.

Es ist anzumerken, daß beim vorschlagsgemäßen Zerstäuber 1 bzw. bei der vorschlagsgemäßen Zähleinrichtung 12 das Antriebselement 17 vorzugsweise zunächst nur axial zum Zählring 14 bzw. Zahnrad 15 hin verlagerbar und erst anschließend gemeinsam mit diesem drehbar ist. Dies wird, wie bereits erwähnt, mittels der ersten Führungsbahn 27 und/oder schiefen Ebene 28 und dem zugeordneten Führungselement 23 erreicht. Jedoch sind hier auch andere konstruktive Lösungen und/oder Abläufe möglich.

Nach Beendigung der Betätigung des Zerstäubers 1 erfolgt die Axialbewegung A in entgegengesetzter Richtung. Insbesondere werden die Führungselemente 23 vom Zählring 14 bzw. Zahnrad 15 axial wieder weiter abgerückt. Dies kann durch eine nicht dargestellte Feder unterstützt werden. Hierdurch wird auch das Antriebselement 17 vom Zahnrad 15 wieder axial abgerückt, so daß diese außer Eingriff gebracht werden. Des weiteren wird dann das Antriebselement 17 im Verlauf der weiteren axialen Rückbewegung der Führungselemente 23 wieder in seine Ausgangslage zurückgedreht. Dies erfolgt wiederum aufgrund des entsprechenden Verlaufs der ersten Führungsbahnen 27 bzw. schiefen Ebenen 28. Der Zerstäuber 1 ist dann zur erneuten Betätigung bereit.

Beim Darstellungsbeispiel weist die Antriebseinrichtung 16 vorzugsweise zwei Führungselemente 23 und/oder erste Führungsbahnen 27, insbesondere auf entgegengesetzten Seiten, auf also zwei kulissenartige bzw. Zwangsführungen. Jedoch kann auch nur eine einzige derartige Führung vorgesehen sein.

Besonders bevorzugt weist die Zähleinrichtung 12 eine Sperre oder Rasteinrichtung 34 zum Verhindern eines Rückdrehens des Zählrings 14 und/oder Zahnrads 15 und/oder zum schrittweisen Einrasten des Zählrings 14 und/oder Zahnrads 15 auf. Die Sperre bzw. Rasteinrichtung 34 weist beim Darstellungsbeispiel vorzugsweise einen elastisch gegen die Zahnung 32 und/oder in axialer Richtung vorgespannten Rastarm 35 auf, der insbesondere von der Führungshülse 32 gebildet, gehalten und/oder an diese angeformt ist und/oder in einer peripheren Ausnehmung 36 des Antriebselements 17 angeordnet ist, wie in Fig. 3 dargestellt. Jedoch sind hier auch andere konstruktive Lösungen möglich. Insbesondere kann auch ein radiales Einrasten und/oder Sperren in einer Drehrichtung vorgesehen sein.

Besonders bevorzugt bildet der Rastarm 35 zusammen mit der Sägezahnung 32 eine Drehsperre, die ein Drehen des Zahnrads 15 nur in eine Drehrichtung - nämlich in die Drehrichtung D zum Weiterzählen - gestattet.

Besonders bevorzugt bildet die Zähleinrichtung 12 eine Baueinheit, die bei der Montage des Zerstäubers 1 in das Gehäuse 5 als Baueinheit einsetzbar ist. Dies vereinfacht die Montage wesentlich.

Die vorschlagsgemäße Zähleinrichtung 12 kann auch bei sonstigen Inhalatoren oder Zerstäubern 1 eingesetzt werden.

### Bezugszeichenliste

| | |
|---|---|
| 1 | Zerstäuber |
| 2 | Fluid |
| 3 | Behälter |
| 4 | Aerosol |
| 5 | Gehäuse |
| 6 | Ventil |
| 7 | Ventilstamm |
| 8 | Düse |
| 9 | Anschlußstück |
| 10 | Mundstück |
| 11 | Gehäuseteil |
| 12 | Zähleinrichtung |
| 13 | Fenster |
| 14 | Zählring |
| 15 | Zahnrad |
| 16 | Antriebseinrichtung |
| 17 | Antriebselement |
| 18 | Zahnung (Antriebselement) |
| 19 | Unterteil |
| 20 | Oberteil |
| 21 | Sichtfenster |
| 22 | Führungsteil |
| 23 | Führungselement |
| 24 | Führungshülse |
| 25 | Öffnung |
| 26 | Vertiefung |
| 27 | erste Führungsbahn |
| 28 | schiefe Ebene |
| 29 | zweite Führungsbahn |
| 30 | Axialnut |
| 31 | Vorsprung |
| 32 | Zahnung (Zahnrad) |
| 33 | axialer Abschnitt |
| 34 | Rasteinrichtung |
| 35 | Rastarm |
| 36 | Ausnehmung |
| 37 | Schulter |
| | |
| A | Axialbewegung |
| D | Drehrichtung |
| H | Hub |
| L | Längsachse |

## Patentansprüche

1. Zerstäuber (1) zur Abgabe eines Aerosols (4),
mit einer Zähleinrichtung (12) zur Zählung von Betätigungen des Zerstäubers (1),
wobei die Zähleinrichtung (12) einen Zählring (14) und eine Antriebseinrichtung (16) mit einem dem Zählring (14) zugeordneten ringförmigen und starren Antriebselement (17) zum Antreiben des Zählring (14) oder eines dem Zählring (14) zugeordneten Zahnrads (15) aufweist,
**dadurch gekennzeichnet,**
**daß** das Antriebselement (17) mit einer axialen Zahnung (18) ausgebildet ist und
eine erste Führungsbahn (27) aufweist, die eine schiefe Ebene (28) bildet oder enthält oder von einem Schraubenbahnabschnitt gebildet wird, und
**daß** die Antriebseinrichtung (16) ein der Führungsbahn (27) zugeordnetes Führungselement (23) zur Umwandlung einer Axialbewegung (A) in eine Drehbewegung (D) zum Antrieb des Zähirings (14) oder Zahnrads (15) aufweist.

2. Zerstäuber nach Anspruch 1, **dadurch gekennzeichnet, daß** das Antriebselement (17) bei Betätigung des Zerstäubers (1) mittels der Führungsbahn (27) und dem Führungselement. (23) axial zum Zählring (14) oder Zahnrad (15) hin verlagerbar und erst anschließend gemeinsam mit diesem drehbar ist.

3. Zerstäuber nach Anspruch 1 oder 2, **dadurch gekennzeichnet, daß** die erste Führungsbahn (27) einen Schlitz oder eine Nut bildet, in den bzw. die das Führungselement (23) eingreift, das zapfenartig ausgebildet ist, und daß die Antriebseinrichtung (16) nur ein Föhrungselement (23) und eine erste Führungsbahn (27) oder zwei Führungselemente (23) und zwei erste Führungsbahnen (27) aufweist.

4. Zerstäuber nach Anspruch 3, **dadurch gekennzeichnet, daß** der Schlitz oder die Nut ausgehend von einer Eingriffsposition des Führungselements (23) bei nicht betätigtem Zerstäuber (1) zunächst geneigt und dann axial verläuft.

5. Zerstäuber nach einem der voranstehenden Ansprüche, **dadurch gekennzeichnet, daß** das Führungselement (23) nur axial bezüglich der Drehrichtung (D) des Zählring (14) verschiebbar, aber nicht drehbar geführt ist.

6. Zerstäuber nach einem der voranstehenden Ansprüche, **dadurch gekennzeichnet, daß** das Führungselement (23) mittels einer zweiten Führungsbahn (29) axial geführt wird.

7. Zerstäuber nach einem der voranstehenden Ansprüche, **dadurch gekennzeichnet, daß** die Zahnung (18) als Sägezahnung ausgeführt ist.

8. Zerstäuber nach einem der voranstehenden Ansprüche, **dadurch gekennzeichnet, daß** der Zählzing (14) oder das Zahnrad (15) mit einer Sägezahnung versehen ist.

9. Zerstäuber nach einem der voranstehenden Ansprüche, **dadurch gekennzeichnet, daß** die Zähleinrichtung (12) eine Sperre oder Rasteinrichtung (34) zum Verhindern eines Rückdrehens des Zählrings (14) oder Zahnrads (15) oder zum schrittweisen Einrasten des Zählrings (14) oder Zahnrads (15) aufweist.

10. Zerstäuber nach einem der voranstehenden Ansprüche, **dadurch gekennzeichnet, daß** die Zähleinrichtung (12) eine Drehbegrenzung zur Begrenzung der Drehung des Antriebselements (17) aufweist.

11. Zerstäuber nach einem der voranstehenden Ansprüche, **dadurch gekennzeichnet, daß** die Zähleinrichtung (12) eine Führungshülse (24) für das Antriebselement (17) aufweist.

12. Zerstäuber nach den Ansprüchen 9 oder 10 und nach Anspruch 11, **dadurch gekennzeichnet, daß** die Führungshülse (24) die Sperre oder Rasteinrichtung (34) oder die Drehbegrenzung bildet, insbesondere durch radialen Eingriff in eine Umfangsöffnung (36) des Antriebselements (17) oder axialen Eingriff in eine Zahnung (32) des Zählrings (14) oder Zahnrads (15).

13. Zerstäuber nach Anspruch 11 oder 12, **dadurch gekennzeichnet, daß** die Führungshülse (24) das Führungselement (23) axial verschieblich führt, aber gegen Drehen sichert.

14. Zerstäuber nach einem der voranstehenden Ansprüche, **dadurch gekennzeichnet, daß** bei Betätigung des Zerstäubers (1) ein Teil des Zerstäubers (1), einen Hub (H) ausführt, der die Axialbewegung (A) bildet und die Antriebseinrichtung (16) antreibt.

15. Zerstäuber nach Anspruch 14, **dadurch gekennzeichnet, daß** die erste Führungsbahn (27) derart ausgelegt ist, daß sich an die schiefe Ebene (28) ein axial verlaufender Abschnitt (33) der Führungsbahn (27) anschließt, so daß nur die anfängliche Axialbewegung, die bereits bei einer teilweisen Betätigung des Zerstäubers entsteht, die Weiterschaltung der Zähleinrichtung (12) bewirkt.

## Claims

1. Nebuliser (1) for dispensing an aerosol (4),
having a counter (12) for counting actuations of the nebuliser (1),
the counter (12) comprising a counting ring (14) and a drive device (16) having an annular rigid drive element (17) associated with the counting ring (14) for driving the counting ring (14) or a gearwheel (15) associated with the counting ring (14),
**characterised in that**
the drive element (17) is embodied with axial teeth (18) and
comprises a first guide track (27) which forms or contains an inclined plane (28) or is formed by a helical track section, and
**in that** the drive device (16) comprises a guide element (23) associated with the guide track (27), for converting an axial movement (A) into a rotary movement (D) for driving the counting ring (14) or gearwheel (15).

2. Nebuliser according to claim 1, **characterised in that** the drive element (17), on actuation of the nebuliser (1), can be moved axially towards the counting ring (14) or gearwheel (15) by means of the guide track (27) and the guide element (23) and only afterwards can it be rotated jointly therewith.

3. Nebuliser according to claim 1 or 2, **characterised in that** the first guide track (27) forms a slot or a groove in which the guide element (23) of peg-like construction engages, and **in that** the drive device (16) comprises only one guide element (23) and a first guide track (27) or two guide elements (23) and two first guide tracks (27).

4. Nebuliser according to claim 3, **characterised in that** the slot or the groove extends initially on a gradient and then axially, starting from a position of engagement of the guide element (23), when the nebuliser (1) is not actuated.

5. Nebuliser according to one of the preceding claims, **characterised in that** the guide element (23) is guided to be movable only axially relative to the direction of rotation (D) of the counting ring (14), but not rotatably.

6. Nebuliser according to one of the preceding claims, **characterised in that** the guide element (23) is guided to be axially movable by means of a second guide track (29).

7. Nebuliser according to one of the preceding claims, **characterised in that** the teeth (18) are in the form of saw teeth.

8. Nebuliser according to one of the preceding claims, **characterised in that** the counting ring (14) or the gearwheel (15) is provided with saw teeth.

9. Nebuliser according to one of the preceding claims, **characterised in that** the counter (12) comprises a barrier or latching device (34) to prevent the counting ring (14) or gearwheel (15) from turning backwards or for the stepwise latching of the counting ring (14) or gearwheel (15).

10. Nebuliser according to one of the preceding claims, **characterised in that** the counter (12) comprises a rotation limiter for limiting the rotation of the drive element (17).

11. Nebuliser according to one of the preceding claims, **characterised in that** the counter (12) comprises a guide sleeve (24) for the drive element (17).

12. Nebuliser according to claims 9 or 10 and according to claim 11, **characterised in that** the guide sleeve (24) forms the barrier or latching device (34) or the rotation limiter, particularly by radial engagement in a circumferential opening (36) in the drive element (17) or axial engagement in teeth (32) of the counting ring (14) or gearwheel (15).

13. Nebuliser according to claim 11 or 12, **characterised in that** the guide sleeve (24) guides the guide element (23) in axially movable manner, but secures it against rotation.

14. Nebuliser according to one of the preceding claims, **characterised in that** on actuation of the nebuliser (1) a part of the nebuliser (1) performs a stroke (H) that forms the axial movement (A) and drives the drive device (16).

15. Nebuliser according to claim 14, **characterised in that** the first guide track (27) is configured such that adjoining the inclined plane (28) is an axially extending section (33) of the guide track (27), so that the initial axial movement, which occurs after merely partial actuation of the nebuliser, is sufficient on its own to advance the counter (12).

## Revendications

1. Pulvérisateur (1) pour distribuer un aérosol (4), comportant élément de comptage (12) pour compter les actionnements du pulvérisateur (1),
l'élément de comptage (12) présentant un anneau de comptage (14) et une unité d'entraînement (16) dotée d'un élément d'entraînement (17) annulaire et fixe associé à l'anneau de comptage (14) pour entraîner l'anneau de comptage (14) ou une roue dentée (15) associée à l'anneau de comptage (14),
**caractérisé en ce que**
l'élément d'entraînement (17) est conçu avec une denture axiale (18) et présente une première piste de guidage (27) qui forme ou contient un plan oblique (28) ou est formé par un segment de trajectoire hélicoïdale, et
l'unité d'entraînement (16) présente un élément de guidage (23) associé à la piste de guidage (27) pour transformer un mouvement axial (A) en un mouvement rotatif (D) pour entraîner l'anneau de comptage (14) ou la roue dentée (15).

2. Pulvérisateur selon la revendication 1, **caractérisé en ce que** l'élément d'entraînement (17) est déplaçable lors de l'actionnement du pulvérisateur (1) au moyen de la piste de guidage (27) et de l'élément de guidage (23) axialement par rapport à l'anneau de comptage (14) ou à la roue dentée (15) et peut ensuite tourner conjointement avec celui-ci.

3. Pulvérisateur selon la revendication 1 ou 2, **caractérisé en ce que** la première piste de guidage (27) forme une fente ou une rainure avec laquelle l'élément de guidage (23) est mise en prise, lequel est conçu comme un tenon, et **en ce que** l'unité d'entraînement (16) présente soit un seul élément de guidage (23) et une seule première piste de guidage (27), soit deux éléments de guidage (23) et deux premières pistes de guidage (27).

4. Pulvérisateur selon la revendication 3, **caractérisé en ce que** la fente ou la rainure est tout d'abord inclinée à partir d'une position de mise en prise de l'élément de guidage (23) lorsque le pulvérisateur (1) n'est pas actionné, puis s'étend axialement.

5. Pulvérisateur selon l'une des revendications précédentes, **caractérisé en ce que** l'élément de guidage (23) n'est déplaçable qu'axialement par rapport à l'axe de rotation (D) de l'anneau de comptage (14) et ne peut être mis en rotation.

6. Pulvérisateur selon l'une des revendications précédentes, **caractérisé en ce que** l'élément de guidage (23) est déplacé axialement au moyen d'une deuxième piste de guidage (29).

7. Pulvérisateur selon l'une des revendications précédentes, **caractérisé en ce que** la denture (18) est conçue en dents de scie.

8. Pulvérisateur selon l'une des revendications précédentes, **caractérisé en ce que** l'anneau de comptage (14) ou la roue dentée (15) est doté(e) d'une denture en dents de scie.

9. Pulvérisateur selon l'une des revendications précédentes, **caractérisé en ce que** l'élément de comptage (12) présente un blocage ou un élément d'encliquetage (34) pour empêcher un retour de l'anneau de comptage (14) ou de la roue dentée (15) ou pour l'enclenchement par palier de l'anneau de comptage (14) ou de la roue dentée (15).

10. Pulvérisateur selon l'une des revendications précédentes, **caractérisé en ce que** l'élément de comptage (12) présente une limite de rotation pour limiter la rotation dé l'élément d'entraînement (17).

11. Pulvérisateur selon l'une des revendications précédentes, **caractérisé en ce que** l'élément de comptage (12) présente une gaine de guidage (24) pour l'élément d'entraînement (17).

12. Pulvérisateur selon les revendications 9 ou 10 et selon la revendication 11, **caractérisé en ce que** la gaine de guidage (24) forme le blocage ou l'élément d'encliquetage (34) ou la limite de rotation, en particulier par mise en prise radiale dans une ouverture périphérique (36) de l'élément d'entraînement (17) ou par mise en prise axiale dans une denture (32) de l'anneau de comptage (14) ou de la rouge dentée (15).

13. Pulvérisateur selon la revendication 11 ou 12, **caractérisé en ce que** la gaine de guidage (24) guide l'élément de guidage (23) axialement de façon coulissante mais l'empêche de tourner.

14. Pulvérisateur selon l'une des revendications précédentes, **caractérisé en ce que**, lors d'un actionnement du pulvérisateur (1), une partie du pulvérisateur (1) effectue une course (H) qui forme le mouvement axial (A) et entraîne l'unité d'entraînement (16).

15. Pulvérisateur selon la revendication 14, **caractérisé en ce que** la première piste de guidage (27) est conçue dé telle sorte qu'un segment (33) de la piste de guidage (27) soit relié au plan oblique (28) de sorte que seul le mouvement axial initial qui se produit déjà lors de l'actionnement partiel du pulvérisateur entraîne la poursuite de l'avancée de l'élément de comptage (12), le segment (33) s'étendant axialement.
